# EUROPÄISCHE PATENTSCHRIFT

(11) **EP 0 775 113 B1**
(45) Veröffentlichungstag und Bekanntmachung des Hinweises auf die Patenterteilung: **11.04.2001**
(21) Anmeldenummer: 95926973.9
(22) Anmeldetag: 22.07.1995
(51) Int. Cl.: C07D 209/44, A61K 31/40, C07D 209/90, C07D 403/06, C07D 409/12

(54) **N-SUBSTITUIERTE AZABICYCLOHEPTAN-DERIVATE ALS NEUROLEPTIKA USW.**
N-SUBSTITUTED AZABICYCLOHEPTANE DERIVATIVES USEFUL AS NEUROLEPTICS
DERIVES D'AZABICYCLOHEPTANE A SUBSTITUTION AZOTE UTILES COMME NEUROLEPTIQUES

(30) Priorität: 04.08.1994 DE 4427647
(43) Veröffentlichungstag der Anmeldung: 28.05.1997
(73) Patentinhaber: BASF AKTIENGESELLSCHAFT, 67056 Ludwigshafen (DE)
(72) Erfinder: STEINER, Gerd, D-67281 Kirchheim (DE); MUNSCHAUER, Rainer, D-67434 Neustadt (DE); HÖGER, Thomas, D-68535 Edingen-Neckarhausen (DE); UNGER, Liliane, D-67065 Ludwigshafen (DE); TESCHENDORF, Hans-Jürgen, D-67373 Dudenhofen (DE)
(86) Internationale Anmeldenummer: EP9502912
(87) Internationale Veröffentlichungsnummer: WO9604245

(56) Entgegenhaltungen:
- WO-A-95/15312
- DE-A- 4 219 973
- DE-A- 4 243 287

## Beschreibung

Die Erfindung betrifft neue N-substituierte Azabicycloheptan-Derivate, deren Herstellung und Verwendung zur Herstellung von Pharmaka.

Es ist bekannt, daß N-substituierte Azabicycloheptan-Derivate überraschende Affinitäten zu Dopamin- und Serotoninrezeptor-Subtypen aufweisen (DE 42 43 287, DE 42 19 973). Hierbei spielen die beobachteten Affinitäten zum D₄-Dopaminrezeptor-Subtyp eine besondere Rolle.

Es wurde nun gefunden, daß N-substituierte 3-Azabicyclo[3.2.0]heptan-Derivate der Formel I 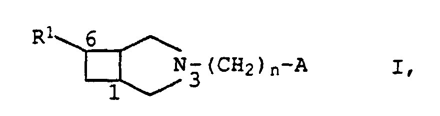 worin
- R¹: eine gegebenenfalls durch Halogenatome mono- oder disubstituierte Naphthyl- oder Phenanthrylgruppe bedeutet,
- n: die Zahl 1, 2, 3 oder 4 bedeutet,
- A: der Rest 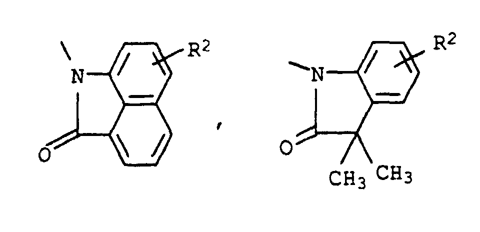 und
- R²: ein Wasserstoffatom, einen Hydroxyrest, eine C₁-C₄-Alkyl-, Nitro- oder Methoxygruppe oder Fluor, Chlor, Brom oder Iod darstellt,
und deren Salze mit physiologisch verträglichen Säuren wertvolle pharmakologische Eigenschaften besitzen.

In der Formel I haben die Substituenten R¹ und R² sowie n vorzugsweise folgende Bedeutungen:
- R¹ :: Naphthyl
- n:: 2
- R²:: Wasserstoff, Fluor, Chlor

Die erfindungsgemäßen Verbindungen der Formel I lassen sich herstellen, indem man eine Verbindung der Formel II

Nu-(CH₂)ₙ-A (II),

in der A und n die angegebenen Bedeutungen haben und Nu eine nukleofuge Abgangsgruppe darstellt, mit einem 3-Azabicyclo[3.2.0]heptan Derivat der Formel III 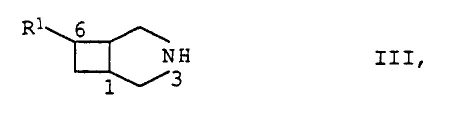 worin
- R¹: eine gegebenenfalls durch Halogenatome mono- oder disubstituierte Naphthyl- oder Phenanthrylgruppe bedeutet,
umsetzt und die so erhaltenen Verbindungen gegebenenfalls in ihre Säureadditionssalze mit physiologisch verträglichen Säuren überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Racemate lassen sich in einfacher Weise durch klassische Spaltung mit optisch aktiven Carbonsäuren, z.B. Weinsäure-Derivaten, in einem inerten Lösungsmittel, z.B. niederen Alkoholen, in die Enantiomeren auftrennen.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedative, Hypnotika, ZNS-Protektiva oder Muskelrelaxantien Verwendung finden. Mehrere der genannten Wirkungsqualitäten können kombiniert bei einer erfindungsgemäßen Verbindung auftreten. Der pharmakologische Wirkungsnachweis erfolgt sowohl in vivo wie auch in vitro, wobei die Substanzcharakterisierung insbesondere durch die teilweise sehr hohe und selektive Affinität zu Rezeptor-Subtypen, vor allem Dopamin D₄-Rezeptoren, möglich ist.

Für die in vivo Charakterisierung der neuen Substanzen wurden folgende Methoden herangezogen:
a) Beeinflussung der Orientierungsmotilität
   Mäuse zeigen in einer neuen Umgebung ein vermehrtes Explorationsverhalten, das sich in einer gesteigerten motorischen Aktivität äußert. Diese motorische Aktivität wird in Lichtschrankenkäfigen für die Zeit von 0 - 30 min nach Einsetzen der Tiere (NMRI-Mäuse, weibl.) in die Käfige gemessen. ED50: Dosis, die die motorische Aktivität im Vergleich zu Placebo-behandelten Kontrollen um 50 % reduziert.
b) Apomorphin-Antagonismus
   Weibliche NMRI-Mäuse erhalten 1,21 mg/kg Apomorphin s.c. Apomorphin führt in dieser Dosis zu einer motorischen Aktivierung, die sich, wenn man die Tiere in Maschendrahtkäfigen hält, in einem permanenten Klettern äußert. Das Klettern wird mit einem Score bewertet (alle 2 min während 30 min):
   0: Tier hat vier Pfoten am Boden
   1: Tier hat zwei Pfoten am Draht
   2: Tier hat vier Pfoten am Draht (klettert).
   Durch Vorbehandlung mit Antipsychotika ist das Kletterverhalten zu hemmen.
   ED50: Dosis, die die Kletteraktivität der Tiere im Vergleich zu Placebo-behandelten Kontrollen um 50 % hemmt.
c) L-5-HTP-Antagonismus
   Weibliche Sprague-Dawley-Ratten erhalten L-5-HTP in einer Dosis von 316 mg/kg i.p. Die Tiere entwickeln darauf ein Erregungssyndrom, von dem Symptome
   - for paw treading und
   - tremor
   mit Hilfe eines Scores (O = nicht vorhanden, 1 = mäßig,
   2 = deutlich ausgeprägt) alle 10 min in der Zeit von 20 bis 60 min nach L-5-HTP-Gabe bewertet werden. Im Mittel wird nach L-5-HTP-Gabe ein Score von 17 erreicht. Die Prüfsubstanzen p.o. werden 60 min vor L-5-HTP gegeben. Als ED50 wird die Dosis errechnet, die im Mittel den Kontrollscore um 50 % vermindert.

Die aufgeführten Methoden sind geeignet, Substanzen als Antipsychotika zu charakterisieren. Mit der Hemmung des L-5-HTP-Syndroms kann eine Serotonin-antagonistische Wirkung aufgezeigt werden, eine Wirkungsqualität, wie sie für die sogenannten atypischen Neuroleptika charakteristisch ist.

Die erfindungsgemäßen Substanzen zeigen in diesen Tests eine gute Wirkung.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II sind bekannt.

Die Substanzen der Formel III lassen sich herstellen, indem man ein Amin der Formel IV 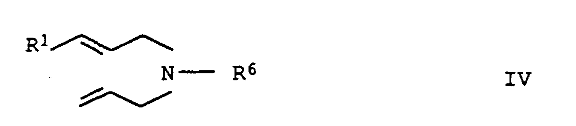 worin R¹ die oben angegebenen Bedeutungen besitzt und R⁶ Wasserstoff, Acetyl, Benzyl oder Trifluoracetyl bedeutet, photochemisch einer [2+2]Cycloaddition unterwirft und anschließend gegebenenfalls eine Acyl- oder Benzylgruppe abspaltet.

Die Photoreaktion gelingt gut in einem inerten Lösungsmittel, vorzugsweise Aceton, bei Temperaturen von 20 bis 80°C. Als Lichtquelle eignet sich besonders gut eine Quecksilberhochdrucklampe. Es ist gegebenenfalls vorteilhaft, die Photocycloaddition in einer Quarzapparatur unter einer Stickstoffatmosphäre gegebenenfalls unter Zusatz von etwa 1 Mol Salzsäure pro Mol Amin durchzuführen.

Die Photocycloaddition verläuft in den meisten Fällen hochdiastereoselektiv zu den bicyclischen Verbindungen III mit der exo-Konfiguration bezüglich R¹ :

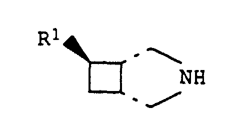

Durch Racematspaltung, z.B. mit optisch aktiven Weinsäure-Derivaten, lassen sich die beiden Enantiomeren rein isolieren.

Die Abspaltung eines Acylrestes (R⁶) erfolgt zweckmäßig durch Verseifung nach bekannten Methoden. Analoges gilt für die Abspaltung des Benzylrestes.

Die Amine der Formel IV sind literaturbekannt oder lassen sich herstellen, indem man entweder einen Aldehyd R¹-CHO mit Vinylmagnesiumchlorid zum Allylalkohol V 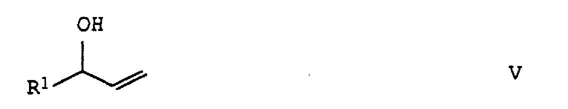 umsetzt, anschließend mit Chlorwasserstoff zum Allylchlorid VI 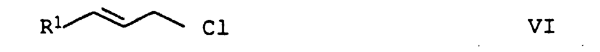 umlagert und zuletzt mit dem entsprechenden Allylamin VII 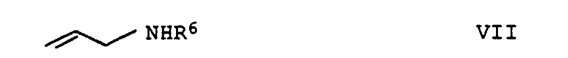 umsetzt, oder man unterzieht einen Zimtaldehyd VIII 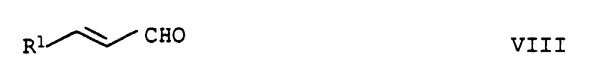 direkt der reduktiven Aminierung mit dem Allylamin VII.

Die folgenden Beispiele dienen zur Erläuterung der Erfindung:

### A) Herstellung der Ausgangsmaterialien

aa) 1-(1-Naphthyl)-allylalkohol
   In einen 2-1-Rührkolben wurden unter Stickstoff 277 ml (360 mM), einer 1,3 M Lösung von Vinylmagnesiumchlorid in Tetrahydrofuran gefüllt. Anschließend wurde unter Rühren und unter Stickstoff innerhalb von 60 min bei 30-35°C eine Lösung von 50 g (320 mM) 1-Naphthaldehyd gelöst in 250 ml Tetrahydrofuran zugegeben. Das Reaktionsgemisch wurde noch 4,5 h unter Stickstoff bei Raumtemperatur gerührt. Danach wurde unter Rühren und Kühlen mit Eis 90 ml gesättigte Ammoniumchlorid-Lösung zugesetzt, abgesaugt und der Filterrückstand dreimal mit 150 ml Tetrahydrofuran gewaschen. Die Filtrate wurden vereinigt, mit Natriumsulfat getrocknet und eingeengt. Man erhielt 58,3 g (99 %) rohes Produkt in Form eines braunen Öls.
ab) 3-(1-Naphthyl)-allylchlorid
   58,3 g (317 mM) 1-(1-Naphthyl)-allylalkohol wurden unter Rühren in 400 ml Dichlormethan gelöst. Anschließend wurde bis zur Sättigung Chlorwasserstoff eingeleitet, wobei die Temperatur auf bis zu 37°C stieg. Es wurde 1 h nachgerührt. Nach dem Waschen mit 200 ml eiskaltem Wasser wurde die organische Phase über Natriumsulfat getrocknet und eingeengt. Man erhielt 59,2 g (92 %) bräunliche Festkörper.
ac) N-Allyl-N-[3-(1-napthyl)-allyl]-amin
   Zu 167 g (2,9 M) Allylamin wurden unter Rückfluß innerhalb von 1 h 59,2 g (0,29 M) 3-(1-Naphthyl)allylchlorid gelöst in 250 ml Toluol zugegeben. Die Mischung wurde 2 h bei Rückflußtemperatur nachgerührt. Anschließend wurde die Reaktionslösung eingeengt, der Rückstand in 250 ml Wasser aufgenommen und mit 50%iger Natronlauge auf pH 12 eingestellt. Die wäßrige Phase wurde mit Dichlormethan extrahiert, die organische Phase über Natriumsulfat getrocknet und eingeengt.
   Ausbeute: 67,6 g (97 %) dunkelbraunes Öl.
ad) exo-6-(1-Naphthyl)-3-azabicyclo[3.2.0]heptan
   50,0 g (193 mM) N-Allyl-N-[3-(1-naphthyl)-allyl] ammoniumchlorid wurden in 1600 ml Aceton gelöst und mit 210 ml 10 %iger Salzsäure versetzt. Die klare gelbe Lösung wurde unter Stickstoff mit einer 700 Watt Quecksilber-Hochdrucklampe in einer Quartzapparatur 4 h bei Raumtempera-. tur bestrahlt. Anschließend wurde die Reaktionslösung eingeengt, der Rückstand in Wasser aufgenommen und mit 50%iger Natronlauge auf pH 12 gestellt. Es wurde 30 min nachgerührt und zweimal mit tert.-Butyl-methylether extrahiert. Die vereinigten organischen Phasen wurden über Natriumsulfat getrocknet und eingeengt.
   Der dunkelbraune ölige Rückstand (43,2 g) wurde in 150 ml Isopropanol gelöst und mit 25,5 g (220 mM) Maleinsäure, gelöst in 220 ml Isopropanol, versetzt. Das ausgefallene Maleinat wurde abgesaugt, mit Isopropanol nachgewaschen und im Vakuumtrockenschrank bei 40°C über Nacht getrocknet. Ausbeute: 43,9 g (67 %) farbloses Pulver, Schmp.: 162-164°C (Maleinat)
Analog lassen sich folgende Substanzen herstellen:
ae) exo-6-(2-Naphthyl)-3-azabicyclo[3.2.0]heptan,
   Schmp.: 145-147°C (Maleinat)
af) exo-6-(5-Chlor-l-naphthyl)-3-azabicyclo[3.2.0]heptan,
ag) exo-6-(9-Phenanthryl)-3-azabicyclo[3.2.0]heptan,
ah) exo-6-(6-Chlor-2-naphthyl)-3-azabicyclo[3.2.0]heptan,
   Schmp. 164-165°C

### B) Herstellung der Endprodukte

### Beispiel 1 (Vergleichsbeispiel)

### N-[2-(exo-6-(1-Naphthyl)-3-azabicyclo[3.2.0]heptan-3-yl)-ethyl]-benzamid

4,0 g (17,8 mM) exo-6-(1-Naphthyl)-3-azabicyclo[3.2.0]heptan in 70 ml Toluol wurden mit 6,6 g (35,2 mM) N-(2-Chlorethyl)benzamid sowie mit 2,5 g (18,1 mM) fein pulverisiertem Kaliumcarbonat und 0,5 g Kaliumjodid versetzt und unter gutem Rühren 6 h unter Rückfluß gekocht. Nach dem Abkühlen engte man am Rotationsverdampfer ein und verteilte den Rückstand zwischen Methylenchlorid und Wasser. Die wäßrige Phase wurde zweimal mit Methylenchlorid nachextrahiert und darauf die organische Phase nach dem Trocknen mit Natriumsulfat eingeengt. Das Rohprodukt (8,9 g) reinigte man durch Säulenchromatographie (Kieselgel, Laufmittel Dichlormethan/Methanol 96/4). Man löste die freie Base (3,0 g) in 100 ml tert.-Butyl-methylether und versetzte unter Eiskühlung mit überschüssiger etherischer Salzsäure. Das ausgefallene Hydrochlorid wurde unter Stickstoff abgesaugt, mit reichlich tert.-Butyl-methylether nachgewaschen und auf der Nutsche im Stickstoffstrom getrocknet. Man isolierte 2,6 g (35 %) Produkt als Hydrochlorid, Schmp. 184-186°C.

Analog lassen sich herstellen: (Vergleichsbeispiele : 2-5, 11-21, 24 und 25)
2. N-[2-(exo-6-(2-Naphthyl)-3-azabicyclo[3.2.0]heptan-3-yl)ethyl]-benzamid, Schmp. 233-235°C (Hydrochlorid),
3. N-[2-(exo-6-(5-Chlor-1-naphthyl)-3-azabicyclo[3.2.0]heptan-3-yl)ethyl]-benzamid,
4. 3-[2-(1-Naphthyl)ethyl]-exo-6-(1-naphthyl)-3-azabicyclo[3.2.0]heptan, Schmp. 227-229°C (Hydrochlorid),
5. 3-[2-(1-Naphthyl)ethyl]-exo-6-(2-naphthyl)-3-azabicyclo[3.2.0]heptan, Schmp. 208-210°C (Hydrochlorid),
6. 1-[2-(exo-6-(l-Naphthyl)-3-azabicyclo[3.2.0]heptan-3-yl)ethyl]-1H-benzo[cd]indol-2-on, Schmp. 174-176°C (Hydrochlorid),
7. 1-[2-(exo-6-(2-Naphthyl)-3-azabicyclo[3.2.0]heptan-3-yl)ethyl]-1H-benzo[cd)indol-2-on, Schmp. 258-260°C (Hydrochlorid),
8. 3,3-Dimethyl-1-[2-(exo-6-(1-naphthyl)-3-azabicyclo[3.2.0]heptan-3-yl) ethyl]-1,3-dihydroindol-2-on,
9. 3,3-Dimethyl-1-[2-(exo-6-(2-naphthyl)-3-azabicyclo[3.2.0]heptan-3-yl)ethyl]-1,3-dihydroindol-2-on, Schmp. 124-125°C,
10. 3,3-Dimethyl-1-[2-(exo-6-(6-chlor-2-naphthyl)-3-azabicyclo[3.2.0]heptan-3-yl)ethyl]-1,3-dihydroindol-2-on,
11. 5-Chlor-N-[2-(exo-6-(1-naphthyl)-3-azabicyclo-[3.2.0]heptan-3-yl)ethyl]-2-thiophencarbonsäureamid,
12. 5-Chlor-N-[2-(exo-6-(2-Naphthyl)-3-azabicyclo[3.2.0]heptan-3-yl)ethyl]-2-thiophencarbonsäureamid.
13. N-[2-(exo-6-(1-Naphthyl)-3-azabicyclo[3.2.0]heptan-3-yl)ethyl]-4-fluor-benzamid,
14. N-[2-(exo-6-(2-Naphthyl)-3-azabicyclo[3.2.0]heptan-3-yl)ethyl]-4-nitro-benzamid,
15. N-[2-(exo-6-(6-Chlor-2-naphthyl)-3-azabicyclo[3.2.0]heptan-3-yl)-ethyl]-benzamid, Schmp. 102-104°C,
16. N-[2-(exo-6-(1-Naphthyl)-3-azabicyclo[3.2.0]heptan-3-yl)ethyl]-4-methoxy-benzamid,
17. N-[2-(exo-6-(2-Naphthyl)-3-azabicyclo[3.2.0]heptan-3-yl)ethyl]-4-hydroxy-benzamid,
18. N-[2-(exo-6-(1-Naphthyl)-3-azabicyclo[3.2.0]heptan-3-yl)ethyl]-3,4-dichlor-benzamid,
19. N-[2-(exo-6-(1-Naphthyl)-3-azabicyclo[3.2.0]heptan-3-yl)ethyl]-naphthalin-1-carbonamid,
20. N-[2-(exo-6-(2-Naphthyl)-3-azabicyclo[3.2.0]heptan-3-yl)ethyl]-naphthalin-1-carbonamid,
21. N-[2-(exo-6-(9-Phenanthryl)-3-azabicyclo[3.2.0]heptan-3-yl)-ethyl]-benzamid, Schmp. 110-112°C (Hydrochlorid),
22. 1-[2-(exo-6-(6-Chlor-2-naphthyl)-3-azabicyclo[3.2.0]heptan-3-yl)-ethyl]-1H-benzo[cd]indol-2-on,
23. 1-[2-(exo-6-(9-Phenanthryl)-3-azabicyclo[3.2.0]heptan-3-yl)-ethyl]-1H-benzo[cd]indol-2-ön,
24. N-[2-(exo-6-(1-Naphthyl)-3-azabicyclo[3.2.0]heptan-3-yl)-ethyl]-isoindolinon,
25. N-[2-(exo-6-(2-Naphthyl)-3-azabicyclo[3.2.0]heptan-3-yl)-ethyl]-isoindolinon.

## Patentansprüche

1. N-substituierte 3-Azabicyclo[3.2.0]-heptan-Derivate der Formel I 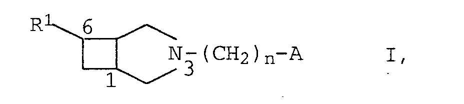 worin
R¹ eine gegebenenfalls durch Halogenatome mono- oder disubstituierte Naphthyl- oder Phenanthrylgruppe bedeutet,
n die Zahl 1, 2, 3 oder 4 bedeutet,
A der Rest 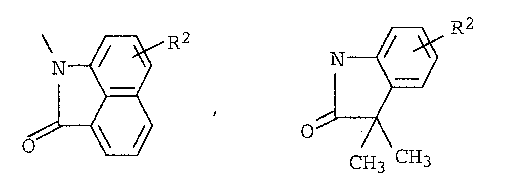 und
R² ein Wasserstoffatom, einen Hydroxyrest, eine C₁-C₄-Alkyl-, Nitro- oder Methoxygruppe oder Fluor, Chlor, Brom oder Iod darstellt,
und deren Salze mit physiologisch verträglichen Säuren.

2. Verbindungen der Formel I gemäß Anspruch 1 zur Verwendung bei der Bekämpfung von Krankheiten.

3. Verfahren zur Herstellung der Verbindungen der Formel I gemäß Anspruch 1, dadurch gekennzeichnet, daß man eine Verbindung der Formel II
Nu-(CH₂)ₙ-A (II),
in der A und n die angegebenen Bedeutungen haben und Nu eine nukleofuge Abgangsgruppe darstellt, mit einem 3-Azabicyclo[3.2.0]heptan Derivat der Formel III 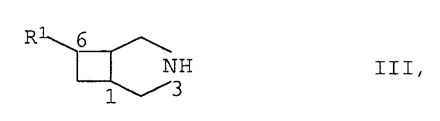 worin
R¹ eine gegebenenfalls durch Halogenatome mono- oder disubstituierte Naphthyl- oder Phenanthrylgruppe bedeutet,
umsetzt und die so erhaltenen Verbindungen gegebenenfalls in ihre Säureadditionssalze mit physiologisch verträglichen Säuren überführt.

4. Verwendung der Verbindungen gemäß Anspruch 1 zur Herstellung eines Arzneimittels zur Behandlung von Psychosen.

## Claims

1. An N-substituted 3-azobicyclo[3.2.0]heptane derivative of the formula I 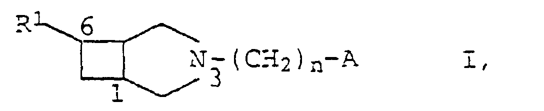 in which
R¹ is a naphthyl or phenanthryl group which is optionally mono- or disubstituted by halogen atoms,
n is the number 1,2,3 or 4,
A is the radical 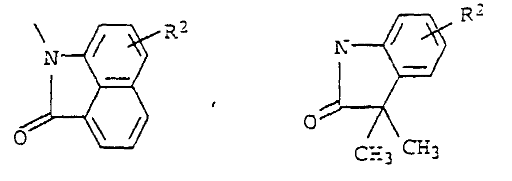 and
R² is a hydrogen atom, a hydroxyl radical, a C₁-C₄-alkyl, nitro or methoxy group or fluorine, chlorine, bromine or iodine,
and salts thereof with physiologically tolerated acids.

2. A compound of the formula I as claimed in claim 1 for use for controlling diseases.

3. The use of a compound as claimed in claim 1 for producing a drug for treating psychoses.

4. A process for preparing a compound of the formula I as claimed in claim 1, which comprises reacting a compound of the formula II
Nu-(CH₂)ₙ-A
in which A and n have the stated meanings, and Nu is a nucleofugic leaving group, with a 3-asabicyclo[3.2.0]heptane derivative of the formula III 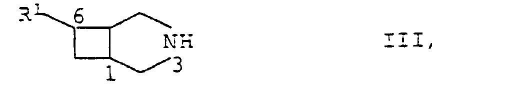 in which
R¹ is a naphthyl or phenanthryl group which is optionally mono- or disubstituted by halogen atoms,
and optionally converting the compound obtained in this way into its acid addition salts with physiologically tolerated acids.

## Revendications

1. Dérivés de 3-azabicyclo[3.2.0]-heptane N-substitués de formule I 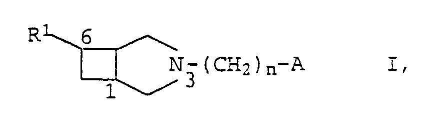 où
R¹ représente un groupe naphtyle ou phénanthryle éventuellement mono- ou disubstitué par des atomes d'halogène,
n désigne le nombre 1, 2, 3 ou 4,
A désigne le reste 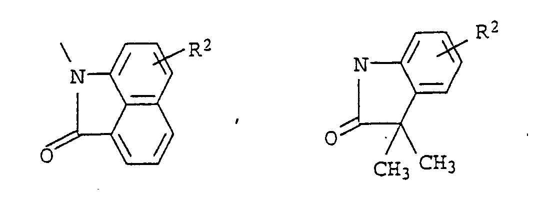
et
R² représente un atome d'hydrogène, un reste hydroxy, un groupe alkyle en C₁-C₄, nitro ou méthoxy, ou le fluor, le chlore, le brome ou l'iode,
et leurs sels avec des acides physiologiquement acceptables.

2. Composés de formule I selon la revendication 1 pour utilisation dans la lutte contre des maladies.

3. Utilisation des composés selon la revendication 1 pour la préparation d'un médicament pour le traitement des psychoses.

4. Procédé pour la préparation des composés de formule I selon la revendication 1, caractérisé par le fait qu'on fait réagir un composé de formule II
Nu- (CH₂)ₙ -A (II)
où A et n ont les significations indiquées, et Nu désigne un groupe séparable nucléofuge, avec un dérivé de 3-azabicyclo[3.2.0]-heptane de formule III 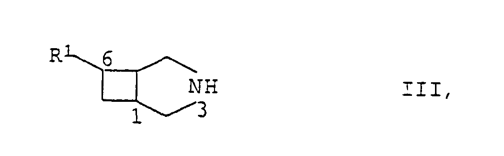
R¹ désigne un groupe naphtyle ou phénanthryle éventuellement mono- ou disubstitué par des atomes d'halogène,
et on convertit éventuellement les composés ainsi obtenus en leurs sels d'addition avec un acide, avec des acides physiologiquement acceptables.
